# EUROPÄISCHE PATENTSCHRIFT

(11) **EP 3 537 169 B1**
(45) Veröffentlichungstag und Bekanntmachung des Hinweises auf die Patenterteilung: **19.02.2025**
(21) Anmeldenummer: 18160421.6
(22) Anmeldetag: 07.03.2018
(51) Int. Cl.: G01R 33/565

(54) **VORRICHTUNG UND VERFAHREN ZUR ERKENNUNG VON SPIKES BEI EINER MAGNETRESONANZMESSUNG**
DEVICE AND METHOD FOR DETECTION OF SPIKES DURING MAGNETIC RESONANCE IMAGING
DISPOSITIF ET PROCÉDÉ DE DÉTECTION DE POTENTIEL D'ACTION LORS D'UNE MESURE EFFECTUÉE PAR RÉSONANCE MAGNÉTIQUE

(43) Veröffentlichungstag der Anmeldung: 11.09.2019
(73) Patentinhaber: Siemens Healthineers AG, 91301 Forchheim (DE)
(72) Erfinder: Nistler, Jürgen, 91056 Erlangen (DE)
(74) Vertreter: Siemens Healthineers Patent Attorneys

(56) Entgegenhaltungen:
- WO-A2-2014/167561
- DE-A1- 19 524 573
- US-A1- 2006 181 278
- US-A1- 2017 108 569

## Beschreibung

Die Erfindung betrifft ein Verfahren und einen Magnetresonanztomographen mit einer Vorrichtung zum Erkennen eines Störsignals, insbesondere von Spikes, während einer Magnetresonanzmessung. Die Vorrichtung weist einen Empfänger mit einem ersten Signalanschluss zum Anschluss einer Lokalspule und einem zweiten Signalanschluss zum Anschluss einer Referenzantenne auf.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, auch als Magnetresonanzsignal bezeichnet, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt. Die erzeugte Darstellung gibt eine räumliche Dichteverteilung der Spins an.

Die empfangenen Magnetresonanzsignale sind sehr schwach, d.h. teilweise nur knapp über der Rauschgrenze. Gleichzeitig haben die Anregungspulse Energien im Kilowattbereich und für die Gradientenfelder fließen Ströme von mehreren hundert Ampere. Durch die hohen Felder und auch indirekt durch mechanischelektrische Wechselwirkungen kann es im Bereich der Patientendurchführungen zur Bildung von elektrischen Potentialen und bei deren Ausgleich zu kleinen Entladungen kommen, die zu kurzen, breitbandigen Störimpulsen, sogenannten Spikes, führen. Über die nachfolgende Bildverarbeitung können dabei aus einzelnen Spikes durch die Fouriertransformation ganze Muster von Artefakten verteilt über das Bild entstehen.

Aus der nicht vorveröffentlichen Patentanmeldung DE 10 2016 223478 ist ein Magnetresonanztomograph bekannt, der physiologische Daten des Patienten erfasst und dabei die Abläufe der Bilderfassung so zeitlich ausrichtet, dass die Erfassung der physiologischen Signale nicht gestört wird.

Aus dem Dokument WO 2014/167561 A2 sind ein Verfahren und ein System zur Echtzeit-Reduktion von Störungen während einer MR-Datenerfassung bekannt. Zumindest eine Antenne ist nahe oder in einem Hohlraum einer üblichen Magnetresonanzvorrichtung angeordnet und steht in Verbindung mit einer üblichen Signalerfassungsvorrichtung. Die Datenerfassung von der Antenne ist synchronisiert mit den Magnetresonanzpulsen. Jedes verbleibende Signal wird als Störung angesehen.

Das Dokument US 2017/0108569 A1 beschreibt ein Magnetresonanzsystem, das neben den Magnetresonanzsignalen simultan mit einer Störerfassungsspule Störsignale sowie Kalibrierungsdaten ohne Anregungspulse erfasst und daraus Kalibrierungs-Störsignale bestimmt.

Aus dem Dokument DE 195 24 573 A1 ist ein MRI-System mit einem Rauschfilter bekannt, welches jedes während eines Scan-Vorgangs erfasste NMR-Signal empfängt und kurzzeitige Rauschimpulse durch Messen des Signalpegels in einem Frequenzband außerhalb der Abbildungsbandbreite detektiert. Eine Ausblendeschaltung unterdrückt das NMR-Signal während jedes Zeitintervalls, in dem ein Rauschimpuls detektiert wird, um den Rauschimpuls vor der Bildrekonstruktion zu entfernen.

Das Dokument US 2006/0181278 A1 betrifft eine Magnetresonanzvorrichtung mit einer Lokalspulenmatrix, eine Pre-Scanning-Einrichtung, die vor der Magnetresonanzaufnahme einen Pre-Scan zumindest eines Teils des Aufnahmebereichs ausführt, und eine Prüfeinheit, die anhand der Lokalspulensignal des Pre-Scans abweichende Lokalspulen erkennt.

Es ist daher eine Aufgabe der vorliegenden Erfindung, die Bildqualität von Magnetresonanztomographien zu verbessern.

Die Aufgabe wird durch den erfindungsgemäßen Magnetresonanztomographen nach Anspruch 1 und das erfindungsgemäße Verfahren nach Anspruch 7 gelöst.

Die Vorrichtung des erfindungsgemäßen Magnetresonanztomographen zum Erkennen eines Spikes während einer Magnetresonanzmessung weist einen ersten Empfänger mit einem ersten Signalanschluss zum Anschluss einer Lokalspule und einem zweiten Empfänger mit einem zweiten Signalanschluss zum Anschluss einer Referenzantenne auf. Die Referenzantenne ist die Körperspule des Magnetresonanztomographen. Die Vorrichtung ist ausgelegt, ein von dem ersten Empfänger über den ersten Signalanschluss empfangenes erstes Signal mit einem von dem zweiten Empfänger über den zweiten Signalanschluss empfangenem zweitem Signal zu vergleichen. Als empfangenes Signal kann dabei beispielsweise das analoge und verstärkte Antennensignal angesehen werden oder ein auf eine Zwischenfrequenz umgesetztes Signal. Denkbar ist auch ein direkt oder nach einer Filterung digitalisiertes Antennensignal. Die Empfänger sind Empfänger bzw. Empfangskanäle des Empfängers des Magnetresonanztomographen, die auch zur Aufbereitung der Magnetresonanzsignale für die Bilderfassung verwendet werden. Die von den Empfängern empfangenen Signale können digital verarbeitet werden.

Der Vergleich kann ein direkter Vergleich der Größe der Amplituden sein, oder einer davon abgeleiteten Größe, wie zum Beispiel der Energie. Eine Mittelung über einen bestimmten Zeitraum ist ebenfalls denkbar. Es kann auch ein Teil des Vergleichs sein, dass die Vorrichtung ausgelegt ist, unterschiedliche Empfindlichkeiten von Lokalspule durch Skalieren eines oder beider Signale mit einem Faktor oder analog durch einen Verstärker auszugleichen. Der Vergleich kann analog beispielsweise durch einen Komparator erfolgen oder bei digitaler Signalverarbeitung durch eine Rechenoperation, wie z.B. Differenz- oder Quotientenbildung. Die Vorrichtung ist ausgelegt, bei einer vorbestimmten Abweichung ein Warnsignal auszugeben. Die vorbestimmte Abweichung kann beispielsweise dann gegeben sein, wenn die Differenz von zweitem Signal und erstem Signal größer ist als 10%, 20%, 50% oder 100% von der Größe des ersten Signals. Es wäre auch denkbar, dass ein Quotient aus dem zweiten Signal zu dem ersten Signal gebildet wird. Ein Überschreiten der vorbestimmten Abweichung kann beispielsweise gegeben sein, wenn der Quotient einen Wert größer als 1,1, 1,3, 1,5 oder zwei annimmt.

Es ist aber auch denkbar, dass komplexere Verfahren zum Einsatz kommen, beispielsweise ein Mustervergleich bzw. eine Autokorrelation zwischen erstem Signal und zweitem Signal. Wird in dem ersten Signal von der Lokalspule das zweite Signal von der Referenzantenne erkannt, ist das Magnetresonanzsignal wahrscheinlich gestört.

Das erfindungsgemäße Verfahren ist in Anspruch 7 definiert**.**

In einem Schritt des Verfahrens wird ein erstes Signal von der Lokalspule über den ersten Signalanschluss des Empfängers empfangen. Unter empfangen ist dabei zu verstehen, dass das Signal zu einer weiteren Verarbeitung zur Verfügung steht, sei es durch eine digitale Signalverarbeitung, wenn das Signal bereits digitalisiert wurde, oder eine analoge Verarbeitung in der erfindungsgemäßen Vorrichtung. Das Signal ist dabei vorzugsweise zeitlich so aufgelöst, dass zumindest die Grundfrequenz eines Spikes (gleich dem Kehrwert der Spikedauer) erfasst wird, beispielsweise indem die Abtastfrequenz das doppelte der Grundfrequenz beträgt bzw. der Frequenzgang des Empfängers zumindest die Grundfrequenz umfasst.

In einem anderen Schritt des Verfahrens wird ein zweites Signal von der Körperspule über den zweiten Signalanschluss des Empfängers empfangen. Dabei gilt das eben gesagte zum Empfangen und der Grundfrequenz.

In einem anderen Schritt des erfindungsgemäßen Verfahrens werden das erste Signal und das zweiten Signal durch die Vorrichtung verglichen. Das Vergleichen erfolgt vorzugsweise durch eine Vergleichseinheit, beispielsweise mit einem analogen Komparator bzw. digital durch Differenz- oder Quotientenbildung oder einen digitalen Komparator. Dabei ist es denkbar, dass als Teil des Vergleichens eines oder beide Signale zunächst verstärkt bzw. skaliert werden, beispielsweise um unterschiedliche Empfindlichkeiten von der Lokalspule und der Referenzantenne auszugleichen.

In einem weiteren Schritt des erfindungsgemäßen Verfahrens prüft die Vorrichtung das Ergebnis des Vergleichs auf eine vorbestimmte Abweichung. Beispielsweise kann das Ergebnis des Vergleichs mit einem analogen oder digitalen Fensterdiskriminator bewertet werden. Denkbar ist auch der Vergleich mit gespeicherten Werten in einer Tabelle oder logische Verknüpfungen von vorbestimmten Ungleichungen unter Verwendung des Vergleichsergebnisses. Wird eine vorbestimmte Abweichung von der Vorrichtung festgestellt, so gibt diese ein Warnsignal aus.

Weitere vorteilhafte Ausführungsformen sind in den Unteransprüchen angegeben.

In einer denkbaren Ausführungsform des erfindungsgemäßen Magnetresonanztomographen ist die Vorrichtung ausgelegt, den Vergleich in Echtzeit auszuführen. Beispielsweise kann die Vorrichtung entsprechende Elemente zur Skalierung, Differenzbildung und Auswertung des ersten Signals und des zweiten Signals aufweise, sei es als verschaltete analoge Bauelemente oder als digitale Signalverarbeitungsresourcen mit entsprechender Programmierung. Dabei kann es in einer Ausführungsform auch als Echtzeit im Sinne der Erfindung angesehen werden, wenn das Vergleichen der Signale und das Erkennen einer Abweichung zwischen zwei Anregungspulsen einer Sequenz erfolgen kann.

Auf vorteilhafte Weise ermöglicht es das Vergleichen und Erkennen in Echtzeit, den aktuell durch einen Spike gestörten Pulszug (engl. puls train) zu wiederholen und mit einem minimalen Zeitaufwand nur den gestörten Teil einer Bilderfassung zu wiederholen anstatt die ganze Bilderfassung und möglicherweise Bildauswertung, wenn das Problem erst durch Artefakte im Bild erkannt wird.

In einer möglichen Ausführungsform des erfindungsgemäßen Magnetresonanztomographen ist die Vorrichtung ausgelegt, den Vergleich nur auszuführen, wenn das erste und/oder das zweite Signal eine vorbestimmte Vorbedingung erfüllen. Beispielsweise kann einer oder die Pegel beider Signale überwacht werden und bei Überschreiten eines vorbestimmten Schwellwertes der Vergleich ausgeführt werden.

Auf vorteilhafte Weise erhöht das Auswerten einer Vorbedingung die Wahrscheinlichkeit für ein korrektes Erkennen eines Störsignals bei gleichzeitiger Reduzierung der Prozessorlast, da beispielsweise bei einer digitalen Vorrichtung der Vergleich nicht permanent erfolgen muss und die Rechenressourcen anderweitig genützt werden können.

In einer denkbaren Ausführungsform des erfindungsgemäßen Magnetresonanztomographen weist die vorbestimmte Abweichung ein Überschreiten eines vorbestimmten Grenzwertes für ein Amplitudenverhältnis des zweiten Signals zu dem ersten Signal auf.

Die Körperspule ist so positioniert, dass diese das Störsignal relativ zu einem Hintergrundrauschen und/oder auch zu dem Magnetresonanzsignal mit höherer Amplitude aufnimmt als die Lokalspule, dadurch dass die Körperspule näher an einer Wand des Patiententunnels angeordnet ist, sodass sie näher an einer potentiellen Entladungsquelle und weiter von der Quelle der Magnetresonanzsignale entfernt ist.

Auf vorteilhafte Weise zeichnen sich dann Störsignale von Spikes durch eine im Verhältnis höhere Amplitude im zweiten Signal gegenüber dem ersten Signal aus und können so leichter identifiziert werden.

In einer möglichen Ausführungsform des erfindungsgemäßen Magnetresonanztomographen weist dieser eine Steuerung in Signalverbindung mit der Vorrichtung auf. Dabei ist die Steuerung ausgelegt, bei Eingehen des Warnsignals von der Vorrichtung den letzten Messzyklus bzw. Pulszug, aufgrund dessen ersten Signalen und zweiten Signalen das Warnsignal erzeugt wurde, zu wiederholen.

Ein gestörtes Magnetresonanzsignal in einem Pulszug bei der Abtastung eines oder mehrerer K-Raum-Punkte hat aufgrund der Algorithmen bei der Bildauswertung Auswirkungen auf das gesamte Bild. Wird jedoch bereits das Störsignal bei der Erfassung eines oder weniger Punkte im K-Raum erkannt, kann dieser einzelne Pulszug wiederholt werden und so vorteilhafter Weise gegenüber einer Wiederholung der ganzen Bilderfassung deutlich Zeit gespart werden.

Der erfindungsgemäße Magnetresonanztomograph weist eine Körperspule auf. Die Körperspule ist dabei als Referenzantenne an den zweiten Signalanschluss der Vorrichtung angeschlossen. Mit anderen Worten, der Magnetresonanztomograph ist ausgelegt, ein mittels der Körperspule empfangenes Signal als zweites Signal an die erfindungsgemäße Vorrichtung weiterzuleiten und dort mit dem ersten Signal zu vergleichen bzw. die anderen in Zusammenhang mit dem zweiten Signal beschriebenen Vorgänge auszuführen.

Die Körperspule weist üblicherweise einen größeren Abstand zu dem Körper des Patienten im Vergleich zu einer Lokalspule auf, während der Abstand zur Wand des Patiententunnels geringer ist. Eine Entladung in der Struktur des Patiententunnels oder häufiger benachbarter Elemente wie HF-Schirm oder Gradientenspule führt daher vorteilhafter Weise bei der Körperspule zu einer größeren Signalamplitude relativ zu einem Magnetresonanzsignal aus dem Körper des Patienten und kann daher insbesondere im Vergleich mit dem ersten Signal der Lokalspule dazu dienen, einen Störimpuls zu identifizieren.

In einer möglichen Ausführungsform des erfindungsgemäßen Magnetresonanztomographen ist dieser dabei ausgelegt, die Körperspule zu verstimmen und ein zweites Signal auf einer Frequenz ungleich der Larmorfrequenz des Magnetresonanztomographen, vorzugsweise in einem benachbarten Frequenzbereich mit einer Frequenzabweichung von mindestens 1%, 5% 10% oder mehr der Larmorfrequenz in der erfindungsgemäßen Vorrichtung zu vergleichen.

Während Magnetresonanzsignale relativ schmalbandig in einem Bereich der Larmorfrequenz liegen, sind gerade Störimpulse von Entladungen relativ breitbandig. Wenn die Referenzantenne verstimmt, d.h. auf eine Resonanzfrequenz ungleich der Larmorfrequenz eingestellt ist, wird das Magnetresonanzsignal weitestgehend in dem zweiten Signal unterdrückt, während die Hüllkurve eines Störimpulses bei dieser Frequenz gegenüber einer Hüllkurve bei der Larmorfrequenz weitgehend unverändert ist. Ein Vergleich einer Hüllkurve des ersten Signals bei der Larmorfrequenz mit einer Hüllkurve eines zweiten Signals bei verstimmter Frequenz hebt dadurch ein mögliches Störsignal auf vorteilhafte Weise weiter gegen das Magnetresonanzsignal ab. Es ist aber auch denkbar, wegen der höheren Empfindlichkeit der Körperspule für E-Felder trotz verstimmter Körperspule den Empfänger auf die Larmorfrequenz abgestimmt zu lassen, wobei die Magnetresonanzsignale aus dem Körper des Patienten wegen des vorwiegenden B-Feldanteils gegenüber dem Spike mit vorwiegender E-Feld-Komponente abgeschwächt werden. Es kann dann ein Empfänger für die Bildgebung ohne Modifikation auch zur Störsignalerkennung genutzt werden.

In einer denkbaren Ausführungsform des erfindungsgemäßen Verfahrens weist das Verfahren weiterhin den Schritt auf, einen Prüfpuls auszusenden. Es ist dabei denkbar, dass der Prüfpuls ein Anregungspuls und ausgelegt ist, Kernspins einer Probe oder eines Patienten anzuregen. Dabei kann die Leistung der Anregungsleistung eines Pulses aus einer Bilderfassungssequenz entsprechen. Vorzugsweise kann die Leistung zur Reduzierung einer SAR Belastung niedriger sein, beispielsweise auf einen Wert um einen Faktor 2, 5, 10 oder geringer. Es ist lediglich erforderlich, dass der Anregungspuls zu einem beliebigen Zeitpunkt nach dem Anregungspuls ein Magnetresonanzsignal der Spins in der Lokalspule und der Referenzantenne erzeugt, dass über einem Rauschpegel liegt.

Es ist aber auch denkbar, dass der Prüfpuls nicht zur Anregung von Kernspins vorgesehen ist, sondern direkt zum Empfang durch die Lokalspule und/oder die Referenzantenne. Dabei kann eine eigene Sendeantenne vorgesehen sein, vorzugsweise in einem Bereich, in dem Spikes entstehen. So empfängt neben der Lokalspule auch die Körperspule ein durch den Prüfpuls erzeugtes Prüfsignal. Es ist aber auch denkbar, dass der Prüfpuls über die Körperspule als Sendeantenne abgestrahlt wird. Da sich die Körperspule in ihrer Geometrie und Eigenschaften im Betrieb kaum verändert, kann hier ein vorbestimmter Referenzwert für das Prüfsignal der Körperspule angenommen werden, dass durch Berechnung oder vorhergehende Messungen gewonnen wird. Die Lokalspule hingegen kann ausgetauscht werden und wird durch den Prüfpuls und das empfangene Prüfsignal vermessen. Vorteilhafter Weise kann durch den direkten Empfang des Prüfpulses als Prüfsignal die Leistung für den Prüfpuls noch geringer in einem Bereich von einigen Watt, Milliwatt oder darunter ausfallen und die SAR-Belastung weiter reduziert werden.

In einem weiteren Schritt wird ein Prüfsignal über die Lokalspule und den ersten Signalanschluss der Vorrichtung empfangen. Sofern der Prüfpuls ein Anregungspuls war und ein Magnetresonanzsignal hervorruft oder der Prüfpuls über eine separate Sendeantenne ausgesendet wurde, wird einem anderen Schritt das Prüfsignal über die Körperspule und den zweiten Signalanschluss der Vorrichtung empfangen.

In einem anderen Schritt wird eine Abhängigkeit des über den ersten Signalschluss empfangenen Prüfsignals zu dem über den zweiten Signalanschluss empfangenen Prüfsignal bestimmt. Die bestimmte Abhängigkeit ist insbesondere dazu geeignet, Unterschiede auf den Empfangswegen zu erkennen, um diese beim Vergleich zu berücksichtigen oder vorher ausgleichen zu können. Sofern der Prüfpuls über die Körperspule ausgesendet und direkt als Prüfsignal von der Lokalspule empfangen wurde, wird dabei für das über den zweiten Signalanschluss empfangene Prüfsignal ein vorbestimmter Wert eingesetzt, der beispielsweise durch eine zu einem früheren Zeitpunkt erfolgte Messung mit einer separaten Sendeantenne ermittelt wurde.

Dabei ist die vorbestimmte Abweichung, die beim Vergleich des ersten Signals von dem zweiten Signal ermittelt und bewertet wird, eine wesentliche Abweichung einer Abhängigkeit des ersten Signals zu dem zweiten Signal von der zuvor bestimmten Abhängigkeit der Prüfsignale. Wurde beispielsweise durch das Prüfsignal festgestellt, dass ein Magnetresonanzsignal aus dem Körper in der Körperspule immer nur eine Signalamplitude von 20% gegenüber dem Signal der Lokalspule aufweist, so kann ein Störsignal, dass in der Körperspule ein Signal mit einer Amplitude von 50% oder mehr gegenüber dem in der Lokalspule erzeugten Signals aufweist, als vorbestimmte Abweichung angesehen werden, indem es mit einem Verhältnis 50:100 wesentlich von dem sonstigen Amplitudenverhältnis 20:100 abweicht.

Indem die Abhängigkeit von über die Körperspule und die Lokalspule empfangenen Signalen anhand eines Anregungspulses in einem Kalibriervorgang bestimmt bzw. ermittelt wird, kann das erfindungsgemäße Verfahren unterschiedliche Geometrien, beispielsweise bei einer veränderten Position oder Art der Lokalspule berücksichtigen und auf diese Weise Störungen zuverlässiger und mit höherer Empfindlichkeit erkennen und deren Folgen in der Bilddarstellung verhindern.

In einer möglichen Ausführungsform des erfindungsgemäßen Verfahrens ist die in dem zuvor beschriebenen Kalibriervorgang bestimmte Abhängigkeit ein Maß für das Verhältnis der Amplituden des über den zweiten Signalanschluss empfangenen Magnetresonanzsignals zu dem über den ersten Signalanschluss empfangenen Magnetresonanzsignals.

Eine wesentliche Abweichung im Sinne der Erfindung liegt dann vor, wenn das vorbestimmte Verhältnis um mehr als 10%, 20% 50% oder 100% überschritten wird.

Durch den zuvor beschriebenen Kalibiervorgang ist es bekannt, wie bzw. mit welcher Amplitude ein Prüfsignal beziehungsweise ein erwünschtes Magnetresonanzsignal aus dem Körper des Patienten von der Lokalspule und der Körperspule empfangen wird. Weicht ein Signal, das über die Lokalspule und die Körperspule empfangen wird, von diesem Amplitudenverhältnis ab, so ist dies ein Hinweis, dass das Signal nicht aus dem Körper des Patienten kommt und damit kein Nutzsignal, sondern ein Störsignal ist. Dabei ist es denkbar, dass bei dem Vergleich das im Kalibriervorgang bestimmte Amplitudenverhältnis der Maßstab für den Vergleich ist oder dass durch eine Skalierung der Signale entsprechend der bei der Kalibrierung ermittelten Amplituden jeweils auf ein Verhältnis von 1:1 normiert wird.

In einer denkbaren Ausführungsform des erfindungsgemäßen Verfahrens ist die Referenzantenne eine Körperspule. Dabei wird die Körperspule in dem Schritt des Empfangens über den zweiten Signalanschluss verstimmt. Beispielsweise kann eine Steuerung des Magnetresonanztomographen eine Kapazität durch einen Schalter in Signalverbindung mit der Körperspule bringen und so die Resonanzfrequenz der Körperspule gegenüber der Larmorfrequenz verschieben, beispielsweise um 2%, 5%, 10% oder mehr. Denkbar sind auch variable Kapazitäten wie PIN-Dioden, bei denen das Maß der Verstimmung über eine angelegte Spannung steuerbar ist.

Die Körperspule weist durch die Nähe zur Wandung des Patiententunnels und der Gradientenspule eine besondere Empfindlichkeit für Störungen in diesem Bereich auf und ermöglicht eine bessere Erkennung von Störsignalen bei gleichzeitigem Empfang über Körperspule und Lokalspule. Die Unterdrückung des Magnetresonanzsignals durch das Verstimmen erleichtert darüber hinaus auf vorteilhafte Weise die Unterscheidung zu dem Magnetresonanzsignal.

In einer möglichen Ausführungsform des erfindungsgemäßen Verfahrens weist das Verfahren weiterhin den Schritt auf, die letzte Magnetresonanzmessung durch den Magnetresonanztomographen zu wiederholen, wenn das Warnsignal von der Vorrichtung eingeht. Als letzte Magnetresonanzmessung wird insbesondere der Messablauf nach dem letzten Hochfrequenz-Anregungspuls für die Kernspins angesehen, sodass die gestörten Daten durch eine identische Messung ohne Störung ersetzt werden können.

Auf vorteilhafte Weise ermöglicht das erfindungsgemäße Verfahren, eine Störung in Echtzeit zu erkennen, sodass nur der gestörte Teil einer Messung wiederholt werden muss, um die gestörten Daten durch ungestörte zu ersetzen und so Fehler in der Bildverarbeitung zu vermeiden.

In einer denkbaren Ausführungsform des erfindungsgemäßen Verfahrens weist das Verfahren weiterhin den Schritt auf, das Störsignal aus der letzten Magnetresonanzmessung durch den Magnetresonanztomographen zu entfernen, wenn das Warnsignal von der Vorrichtung eingeht. Beispielsweise ist es denkbar, dass die Daten, die unmittelbar während der Störung erfasst wurden, gelöscht oder durch einen konstanten Wert ersetzt werden. Denkbar ist auch eine Interpolation. Vorzugsweise weist dabei das Warnsignal eine Information über den Beginn und das Ende der Störung auf.

Denkbar wäre es aber auch, dass durch Korrelation von erstem Signal und zweitem Signal ein Anteil des Störsignals in dem ersten Signal bestimmt und aus dem ersten Signal entfernt wird, beispielsweise indem das Störsignal von dem Empfänger und/oder der Steuerung des Magnetresonanztomographen entsprechend skaliert und subtrahiert wird.

Auf vorteilhafte Weise ermöglicht es der Empfang mit Lokalspule und Körperspule, das Störsignal von dem Magnetresonanzsignal zu unterscheiden und zu separieren, sodass auch ohne zusätzliche Messung die Daten korrigiert werden und Artefakte entsprechend reduziert werden können.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
Fig. 1 eine beispielhafte schematische Darstellung eines erfindungsgemäßen Magnetresonanztomographen
Fig. 2 eine schematische Darstellung der Vorrichtung zum Erkennen von Spikes des erfindungsgemäßen Magnetresonanztomographen;
Fig. 3 einen schematischen Ablaufplan eines erfindungsgemäßen Verfahrens zum Erkennen von Spikes,

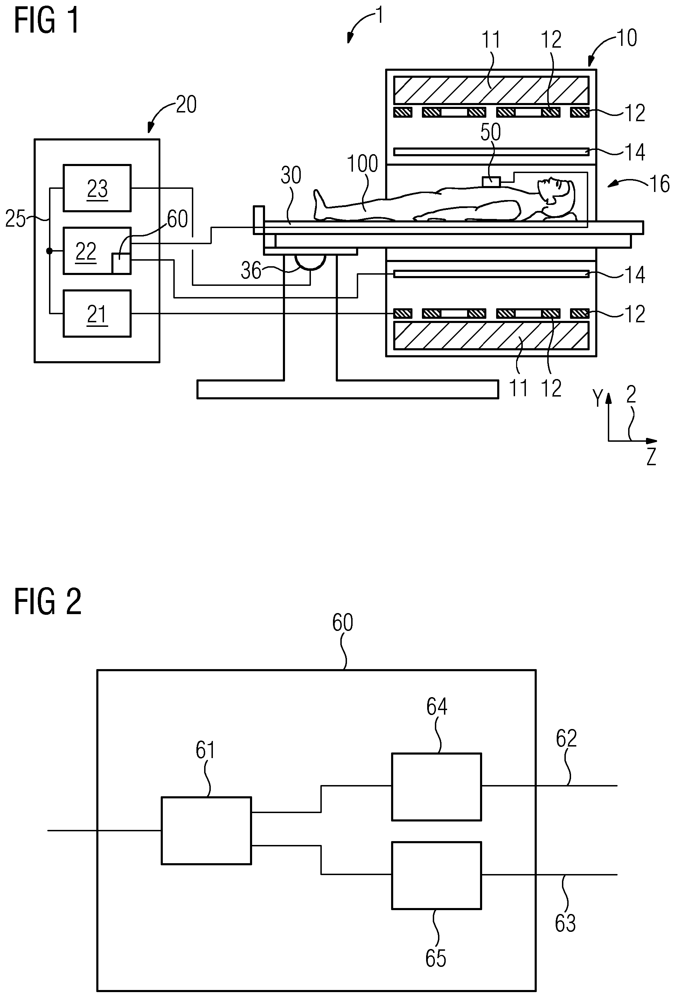

Fig. 1 zeigt eine schematische Darstellung einer Ausführungsform eines erfindungsgemäßen Magnetresonanztomographen 1 mit einer Vorrichtung 60 zum Erkennen von Spikes.

Die Magneteinheit 10 weist einen Feldmagneten 11 auf, der ein statisches Magnetfeld B0 zur Ausrichtung von Kernspins von Proben bzw. Patienten 100 in einem Aufnahmebereich erzeugt. Der Aufnahmebereich ist in einem Patiententunnel 16 angeordnet, der sich in einer Längsrichtung 2 durch die Magneteinheit 10 erstreckt. Ein Patient 100 ist mittels der Patientenliege 30 und der Verfahreinheit 36 der Patientenliege 30 in den Aufnahmebereich verfahrbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Feldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können. Die Magneteinheit 10 weist ebenfalls eine Körperspule 14 auf, die dazu ausgelegt ist, ein über eine Signalleitung 33 zugeführtes Hochfrequenzsignal in das Untersuchungsvolumen abzustrahlen und von dem Patient 100 emittierte Resonanzsignale zu empfangen und über eine Signalleitung abzugeben. Bevorzugter Weise wird aber die Körperspule 14 für das Aussenden des Hochfrequenzsignals und/oder das Empfangen durch Lokalspulen 50 ersetzt, die in dem Patiententunnel 16 nahe am Patient 100 angeordnet sind. Es ist aber auch denkbar, dass die Lokalspule 50 zum Senden und Empfangen ausgelegt ist.

Eine Steuereinheit 20 versorgt die Magneteinheit 10 mit den verschiedenen Signalen für die Gradientenspulen 12 und die Körperspule 14 und wertet die empfangenen Signale aus. Eine Magnetresonanztomographen-Steuerung 23 koordiniert dabei die Untereinheiten.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die einzelnen Einheiten sind über einen Signalbus 25 untereinander verbunden.

Das von Hochfrequenzeinheit 22 erzeugte Hochfrequenzsignal wird über eine Signalverbindung der Körperspule 14 zugeführt und in den Körper des Patienten 100 ausgesendet, um dort die Kernspins anzuregen. Denkbar ist aber auch ein Aussenden des Hochfrequenzsignals über eine oder mehrere Lokalspulen 50. Die Lokalspule 50 empfängt dann vorzugsweise ein Magnetresonanzsignal aus dem Körper des Patienten 100, denn aufgrund des geringen Abstandes ist das Signal-zu-Rauschverhältnis (SNR) der Lokalspule 50 besser als bei einem Empfang durch die Körperspule 14. Das von der Lokalspule 50 empfangene MR-Signal wird in der Lokalspule 50 aufbereitet und an die Hochfrequenzeinheit 22 des Magnetresonanztomographen 1 zur Auswertung und Bilderfassung weitergeleitet. Vorzugsweise wird dazu die Signalverbindung 33 genutzt, es ist aber beispielsweise auch eine drahtlose Übertragung denkbar.

Die Vorrichtung 60 zum Erkennen von Spikes ist nachfolgend zur Fig. 2 näher beschrieben. Die in Fig. 2 dargestellte Vorrichtung 60 weist einen ersten Signalanschluss 62 auf, der mit der Lokalspule 50 in Signalverbindung steht und ein empfangenes Magnetresonanzsignal von der Lokalspule 50 empfängt. Das Magnetresonanzsignal kann dabei analog oder digital sein. Weiterhin weist die Vorrichtung 60 einen zweiten Signalanschluss 63 auf, der mit der Körperspule 14 als Referenzantenne, in Signalverbindung steht.

Das Signal der Referenzantenne kann wie das Signal der Lokalspule 50 analog oder digital sein und durch eine Signalverarbeitung vorbearbeitet, beispielsweise in einen anderen Frequenzbereich umgesetzt oder gefiltert sein.

Die Antennensignale am ersten Signalanschluss 62 und am zweiten Signalanschluss 63 werden von einem ersten Empfänger 64 und einem zweiten Empfänger 65 aufbereitet. Sind die Antennensignale beispielsweise analog, so kann der Empfänger 64, 65 eine Verstärker- und eine Filterstufe aufweisen, um die Signale zu verstärken und von Störungen abseits der Frequenz des Magnetresonanzsignals zu befreien. Denkbar ist es auch, dass die Signale auf eine Zwischenfrequenz umgesetzt werden und/oder demoduliert und/oder digitalisiert. Sind die Antennensignale bereits digitalisiert, so kann eine digitale Signalbearbeitung mit entsprechenden Filtern bzw. Funktionen erfolgen. Grundsätzlich können die Empfänger 64, 65 exklusiv für die Vorrichtung 60 vorgesehen sein. Vorzugsweise werden aber Empfänger bzw. Empfangskanäle der Hochfrequenzeinheit 22 genutzt, die auch für die Bilderfassung eingesetzt werden. Insbesondere für die Signale der Lokalspule 50 können die gleichen Signale des ersten Empfängers 64 in Signalverbindung mit der Lokalspule 50 sowohl zum Erkennen eines Spikes als auch zu Bilderfassung genutzt werden.

Weiterhin weist die Vorrichtung 60 eine Vergleichseinheit 61 auf, die ausgelegt ist, die über den ersten Signalanschluss 62 eingehenden Signale und die über den zweiten Signalanschluss 63 eingehen miteinander in Beziehung zu setzen.

Vorzugsweise ist die Vorrichtung 60 ausgelegt, den Vergleich in Echtzeit auszuführen. Dies kann beispielsweise durch eine analoge Komparatorschaltung oder eine digitale Signalverarbeitung geschehen.

Insbesondere eine digitale Signalverarbeitung ist in der Lage, die Signale nach einem komplexen Kriterium zu bewerten, beispielsweise, ob diese in einem bestimmten Muster wie Anstiegsgeschwindigkeit, Verlauf, Dauer, Frequenzverteilung oder ähnlichem einem Spike entsprechen. Eine digitale Signalverarbeitung kann derartige Muster gespeichert haben, aber auch ein analoger Hochpass- oder Bandpass-Filter ist in der Lage eine Anstiegsgeschwindigkeit oder Frequenzverteilung zu unterscheiden.

Aufgrund der Bewertung nach unterschiedlichen Kriterien kann die Vorrichtung 60 dann auch ausgelegt sein, einen Vergleich nur auszuführen, wenn das erste und/oder das zweite Signal eine vorbestimmte Vorbedingung erfüllen. Beispielsweise können Diskriminatoren in der Vergleichseinheit 61 nur Signale zum eigentlichen Vergleich durchlassen, die eine bestimmte Mindestamplitude überschreiten, um Fehlauslösungen durch Rauschen zu vermeiden. Die Diskriminatoren könnten aber auch zusätzlich oder alternativ eine Filterfunktion aufweisen, um nur vorbestimmte spezifische Frequenzverteilungen durchzulassen.

Eine bevorzugtes Vergleichskriterium für die Vergleichseinheit 61 kann beispielsweise ein zur Erkennung eines Spikes mindestens zu erzielendes Amplitudenverhältnis von einem zweiten Signal am zweiten Signalanschluss 63 von der Körperspule 14 zu einem ersten Signal am ersten Signalanschluss 62 der Lokalspule 50 sein, denn für einen Spike in der Nähe der Körperspule 14 ist ein deutlich stärkeres Signal über den mit der Körperspule 14 zweiten Signalanschluss 63 zu erwarten als von einer weiter entfernten Lokalspule 50 an dem ersten Signalanschluss 62.

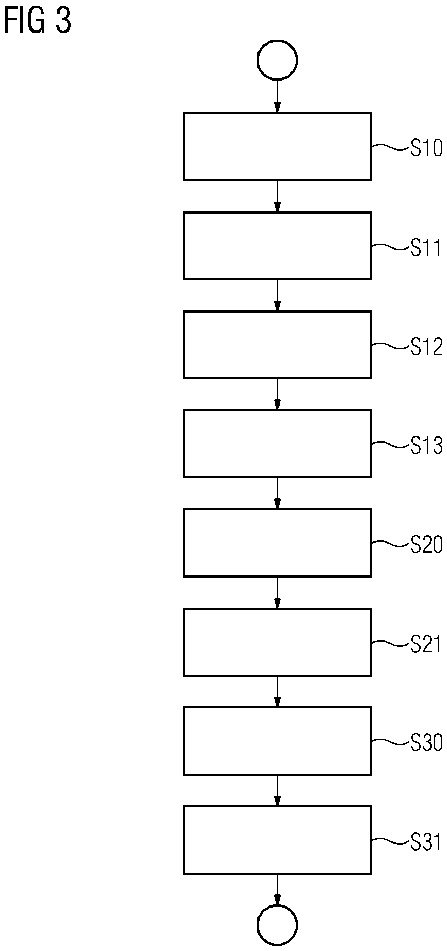

In Fig. 3 ist ein schematisches Ablaufdiagramm für eine mögliche Ausführungsform eines erfindungsgemäßen Verfahrens zum Erkennen eines Spikes bei einer Magnetresonanzmessung mit einem Magnetresonanztomographen 1 dargestellt.

In einem Schritt S20 wird ein erstens Signal von der Lokalspule 50 über den ersten Signalanschluss 62 der Vorrichtung über den ersten Empfänger 64 empfangen. Das Empfangen kann dabei beispielsweise auch ein Filtern, Umsetzen in eine Zwischenfrequenz oder ein Digitalisieren umfassen.

In einem Schritt S21 wird ein zweites Signal von der Körperspule als Referenzantenne über den zweiten Signalanschluss 63 der Vorrichtung empfangen. Vorzugsweise werden die Schritte S2C und S21 gleichzeitig und auch in Echtzeit ausgeführt. Dabei gilt das unter S20 zum Empfangen gesagte ebenfalls in Schritt S21.

In einem Schritt S30 werden das erste Signal und das zweite Signal durch die Vorrichtung, vorzugsweise die Vergleichseinheit 61 verglichen. Das Vergleichen kann dabei beispielsweise das Bilden einer Differenz und/oder Quotienten aus erstem Signal und zweitem Signal umfassen, aber auch andere logische oder arithmetische Funktionen sind denkbar.

In einem Schritt S31 wird ein Warnsignal durch die Vorrichtung an den Magnetresonanztomographen ausgegeben, wenn bei dem Vergleich eine vorbestimmte Abweichung erkannt wird. Der beim Vergleich gewonnene Wert kann beispielsweise mit einem vorbestimmten Schwellwert verglichen werden und bei Überschreiten das Warnsignal ausgegeben werden. Beispielsweise kann beim Vergleich ein Quotient oder eine Differenz aus dem Ausgangssignal des zweiten Empfängers in Signalverbindung mit der Referenzantenne und des ersten Empfängers in Verbindung mit der Lokalspule 50 gebildet werden. Wenn der Quotient für Magnetresonanzsignale aufgrund der Geometrie und unterschiedlicher Empfindlichkeiten bei Magnetresonanzsignalen aus dem Körper zum Beispiel den Wert 0,5 hat, kann ein Schwellwert von 0,7 vorgesehen sein. Nimmt der Quotient dann den Wert 0,8 oder größer an, weil ein Spike in der Körperspule 14 als Referenzantenne ein stärkeres Signal als in der Lokalspule 50 erzeugt, so wird der vorbestimmte Schwellwert überschritten und eine Abweichung erkannt.

In einer möglichen Ausführungsform des erfindungsgemäßen Verfahrens ist es dabei denkbar, dass auch eine Kalibrierung der Vorrichtung 60 erfolgt, um unterschiedliche Empfindlichkeiten auszugleichen, insbesondere bei der Verwendung unterschiedlicher Lokalspulen 50.

Dazu wird in einem Schritt S10 ein Prüfpulspuls ausgesendet. Dabei gibt es zwei unterschiedliche Ausführungsformen des erfindungsgemäßen Verfahrens.

In einer Ausführungsform ist der Prüfpuls ein Anregungspuls für Kernspins einer Probe bzw. des Patienten 100 in dem Patiententunnel 16. Die Kernspins erzeugen wiederum ein Magnetresonanzsignal, das in einem Schritt S11 über die Lokalspule 50 und den ersten Signalanschluss 62 und in einem Schritt S12 über die Referenzantenne und den zweiten Signalanschluss 63 empfangen wird, um ein Amplitudenverhältnis bzw. eine relative Sensitivität der Lokalspule 50 zu der Referenzantenne ermitteln. Da die Anregung und der Empfang des Magnetresonanzsignals als Prüfsignal zeitversetzt erfolgt, kann als Sendeantenne für den Prüfpuls wie auch als Referenzantenne für das Prüfsignal in Form des Magnetresonanzsignals die Körperspule 14 selbst dienen. Auch wird hier unmittelbar der unterschiedliche und veränderliche Abstand zwischen Patient 100 zu Lokalspule 50 und Körperspule 14 berücksichtigt, wie er nachfolgend auch bei den Magnetresonanzsignalen für die Bildgebung vorliegt. Allerdings sind zur Anregung der Kernspins wesentlich höhere Leistungen erforderlich, sodass der Prüfpuls das SAR-Budget stärker belastet, auch wenn er wegen des geringeren erforderlichen Signal-zu-Rauschverhältnisses schwächer als bei der Bildgebung ausfallen kann, beispielsweise mit einer Leistung kleiner als 1000 W, 500 W, 200 W oder 100 W.

In einer anderen Ausführungsform wird der Prüfpuls unmittelbar und gleichzeitig als Prüfsignal empfangen. Es ist dazu entweder denkbar, dass der Prüfpuls über eine separate Sendeantenne abgestrahlt wird, sodass die Lokalspule 50 in einem Schritt S11 über den ersten Signalanschluss 62 bzw. die Körperspule 14 in einem Schritt S12 über den zweiten Signalanschluss 63 diesen Prüfpuls als Prüfsignal unmittelbar und gleichzeitig empfangen können. Bei der nachfolgenden Bestimmung der Abhängigkeit ist dann die Position der Sendeantenne zu berücksichtigen. Ist die Sendeantenne in der Nähe des Entstehungsortes von Spikes nahe zu der Wand des Patiententunnels und zu der Gradientenspule, so entsprechen die empfangenen Signalstärken des Prüfsignals eher dem Verhältnis der Signalstärken für einen Spike, während bei einer Anordnung näher zu der Lage des Patienten 100 eher dem Verhältnis der Signalstärken für das Magnetresonanzsignal entsprechen. Für den letzteren Fall ist es denkbar, dass die Lokalspule 50 eine Sendeantenne aufweist.

Es ist auch denkbar, dass der Prüfpuls über die Körperspule 14 ausgesendet wird, wobei ein gleichzeitiger Empfang über die Körperspule 14 dann nicht möglich ist. Es ist daher erforderlich für das Prüfsignal der Körperspule 14 als Referenzantenne einen vorbestimmten, beispielsweise während einer vorhergehenden Messung in einem Schritt S12 über die Referenzantenne und den zweiten Signalanschluss 63 empfangenen oder durch Berechnung gewonnenen Wert einzusetzen.

In beiden Fällen kann die Leistung des Prüfpulses vorteilhafter Weise auf weniger als 0,01 Watt, 0,1 Watt, 1 Watt oder 10 Watt reduziert werden, sodass der Prüfpuls im SAR-Budget nicht zu berücksichtigen ist.

In einem Schritt S13 wird eine Abhängigkeit von dem über den ersten Signalschluss empfangenen Prüfsignal zu dem über den zweiten Signalanschluss empfangenen Prüfsignal bestimmt. Sie kann beispielsweise ein Verhältnis oder eine Differenz der Amplituden oder Leistung der Prüfsignale sein, absolut oder gemittelt über die Signale. Erfolgt die Kalibrierung durch einen Prüfpuls mit einer Quelle in der Nähe der Referenzantenne, so kann eine Abhängigkeit beispielsweise auch durch einen Kehrwert des Verhältnisses gebildet werden oder durch einen Schwellwert, der einem Mittelwert der beiden Signale entspricht.

Die vorbestimmte Abweichung in Schritt S31 ist dann vorzugsweise eine wesentliche Abweichung einer Abhängigkeit des ersten Signals zu dem zweiten Signal von der in Schritt S13 bestimmten Abhängigkeit. Vorzugsweise ist die Abhängigkeit auf ein Magnetresonanzsignal bezogen, sodass beispielsweise ein Signal, dessen Amplitudenverhältnis davon abweicht, indem das empfangene Signal für die Körperspule 14 mit Referenz auf die bzw. Gewichtung mit der ermittelten Abhängigkeit stärker ist als das von der Lokalspule 50 empfangene Signal, als Spike erkannt wird.

Die Abhängigkeit kann in einer Ausführungsform ein Maß für das Verhältnis der Amplituden des über den zweiten Signalanschluss 63 empfangenen Prüfsignals zu dem über den ersten Signalanschluss 62 empfangenen Prüfsignals sein. Eine wesentliche Abweichung im Sinne der Erfindung liegt dann beispielsweise vor, wenn das vorbestimmte Verhältnis um mehr als 10%, 20% 50% oder 100% überschritten wird.

Es kann in dem Schritt S21 und/oder S12 des Empfangens über den zweiten Signalanschluss vorgesehen sein, dabei, d.h. während des Empfangens des Signals bzw.

Prüfsignals, die Körperspule 14 zu verstimmen. Ein Störsignal bzw. Spike weist üblicherweise ein Frequenzspektrum auf, das breiter als ein Magnetresonanzsignal ist. Indem die Körperspule 14 zum Empfang eines Störsignals und/oder beim Kalibrieren in Schritt S12 gegenüber der Larmorfrequenz um mehr als 10 kHz, 100 kHz oder 1 MHz verstimmt ist, kann das Störsignal gegenüber einem Magnetresonanzsignal hervorgehoben und leichter erkannt werden. Dies gilt auch, wenn der zweite Empfänger 65 selbst auf der Larmorfrequenz abgestimmt verbleibt, denn die Körperspule 14 weist eine erhöhte Sensitivität für E-Felder auf, die bei einem Spike im Nahbereich überwiegen und ihn gegenüber den B1-Feldern der MR-Signale aus dem Körper des Patienten 100 von der Körperspule 14 hervorheben.

In einem Schritt S40 einer Ausführungsform des erfindungsgemäßen Verfahrens wird die letzte Magnetresonanzmessung durch den Magnetresonanztomographen 1 wiederholt, wenn das Warnsignal von der Vorrichtung 60 eingeht. Beispielsweise steht die Vorrichtung 60 über eine Signalverbindung mit der Steuereinheit 20 des Magnetresonanztomographen 1 in Signalverbindung, über die das Warnsignal übertragen werden kann. Erhält die Steuereinheit 20 dieses Warnsignal, so wiederholt sie vorzugsweise den letzten Pulszug, d.h. den Ablauf der Bilderfassung nach dem letzten Anregungspuls, um störungsfreie Messdaten zur Bildrekonstruktion ohne Artefakte durch Spikes zu erhalten.

In einer anderen möglichen Ausführungsform ist die Steuereinheit 20 dazu ausgelegt, das Störsignal aus der letzten Magnetresonanzmessung zu entfernen, wenn das Warnsignal von der Vorrichtung 60 eingeht. Beispielsweise ist es denkbar, dass die Steuereinheit 20 Messdaten für die Dauer der Störung durch Interpolation simuliert. Es wäre auch denkbar, dass aus dem Magnetresonanzsignal der Referenzantenne das Störsignal extrahiert wird und z.B. durch Skalierung und Subtraktion oder komplexere Verfahren aus den Messdaten im Wesentlichen entfernt wird.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen, der durch die angehängten Ansprüche definiert ist.

## Patentansprüche

1. Magnetresonanztomograph mit einer Vorrichtung zum Erkennen eines Spikes während einer Magnetresonanzmessung, wobei die Vorrichtung aufweist:
einen ersten Empfänger (64) mit einem ersten Signalanschluss (62) zum Anschluss einer Lokalspule (50) und einem zweiten Empfänger (65) mit einem zweiten Signalanschluss (63) zum Anschluss einer Referenzantenne,
wobei die Vorrichtung (60) ausgelegt ist, ein von dem ersten Empfänger (64) über den ersten Signalanschluss (62) empfangenes erstes Signal mit einem von dem zweiten Empfänger (65) über den zweiten Signalanschluss (63) empfangenem zweitem Signal mittels einer Vergleichseinheit (61) zu vergleichen und bei einer vorbestimmten Abweichung ein Warnsignal auszugeben,
wobei der Magnetresonanztomograph eine Körperspule und eine Lokalspule aufweist,
und die Lokalspule an den ersten Signalanschluss angeschlossen ist,
**dadurch gekennzeichnet, dass** die Körperspule dabei als Referenzantenne an den zweiten Signalanschluss der Vorrichtung angeschlossen ist.

2. Magnetresonanztomograph nach Anspruch 1, wobei die Vorrichtung (60) ausgelegt ist, den Vergleich in Echtzeit auszuführen.

3. Magnetresonanztomograph nach Anspruch 1 oder 2, wobei der Vergleich einen Mustervergleich zwischen erstem Signal und zweitem Signal aufweist.

4. Magnetresonanztomograph nach einem der vorhergehenden Ansprüche, wobei die Vorrichtung (60) ausgelegt ist, den Vergleich nur auszuführen, wenn das erste und/oder das zweite Signal eine vorbestimmte Vorbedingung erfüllen.

5. Magnetresonanztomograph nach einem der vorhergehenden Ansprüche, wobei die vorbestimmte Abweichung ein Überschreiten eines vorbestimmten Grenzwertes für ein Amplitudenverhältnis des zweiten Signals zu dem ersten Signal aufweist.

6. Magnetresonanztomograph nach einem der vorhergehenden Ansprüche, mit einer Steuereinheit (20) in Signalverbindung mit der Vorrichtung, wobei die Steuereinheit (20) ausgelegt ist, bei Eingehen des Warnsignals von der Vorrichtung (60) den Ablauf einer Bilderfassung nach einem letzten Anregungspuls , aufgrund deren ersten Signalen und zweiten Signalen das Warnsignal erzeugt wurde, zu wiederholen.

7. Verfahren zum Erkennen eines Spikes bei einer Magnetresonanzmessung mit einem Magnetresonanztomographen (1), wobei der Magnetresonanztomograph (1) eine Lokalspule, eine Referenzantenne und eine Vorrichtung (60) zum Erkennen eines Spikes während einer Magnetresonanzmessung mit einem ersten Empfänger (64) mit einem ersten Signalanschluss (62) in Signalverbindung mit der Lokalspule (50) und einem zweiten Signalanschluss (63) in Signalverbindung mit der Referenzantenne aufweist,
wobei der Magnetresonanztomograph eine Körperspule aufweist,
wobei die Körperspule dabei als Referenzantenne an den zweiten Signalanschluss der Vorrichtung angeschlossen ist,
wobei das Verfahren die Schritte aufweist:
(S20) Während einer Magnetresonanzmessung Empfangen eines ersten Signals von der Lokalspule (50) über den ersten Signalanschluss (62) des ersten Empfängers (64);
(S21) Empfangen eines zweiten Signals von der Körperspule über den zweiten Signalanschluss (63) des zweiten Empfängers (65);
(S30) Vergleichen des ersten Signals und des zweiten Signals durch die Vorrichtung (60);
(S31) Ausgeben eines Warnsignals durch die Vorrichtung (60) an den Magnetresonanztomographen (1), wenn bei dem Vergleich eine vorbestimmte Abweichung erkannt wird.

8. Verfahren nach Anspruch 7, wobei das Verfahren weiter die Schritte zum Kalibrieren aufweist, wobei die Schritte zum Kalibrieren vor den Schritten des Anspruchs 7 ausgeführt werden:
(S10) Aussenden eines Prüfpulses;
(S11) Empfangen eines Prüfsignals über die Lokalspule (50) und den ersten Signalanschluss (62) der Vorrichtung (60);
(S12) Empfangen des Prüfsignals über die Referenzantenne und den zweiten Signalanschluss (63) der Vorrichtung (60);
(S13) Bestimmen einer Abhängigkeit von dem über den ersten Signalschluss (62) empfangenen Prüfsignal zu dem über den zweiten Signalanschluss (63) empfangenen Prüfsignal;
wobei die vorbestimmte Abweichung in Schritt (S31) eine wesentliche Abweichung einer Abhängigkeit des ersten Signals zu dem zweiten Signal von der in Schritt (S13) bestimmten Abhängigkeit ist.

9. Verfahren nach Anspruch 8, wobei die bestimmte Abhängigkeit ein Maß für das Verhältnis der Amplituden des über den zweiten Signalanschluss (63) empfangenen Prüfsignals zu dem über den ersten Signalanschluss (62) empfangenen Prüfsignal ist und eine wesentliche Abweichung vorliegt, wenn das vorbestimmte Verhältnis um mehr als 10%, 20% 50% oder 100% überschritten wird.

10. Verfahren nach Anspruch 8 oder 9, wobei der Schritt des Empfangens über den zweiten Signalanschluss (63) den Teilschritt aufweist, die Körperspule (14) zu verstimmen.

11. Verfahren nach einem der Ansprüche 7 bis 10, wobei das Verfahren weiterhin die Schritte aufweist:
Während der Magnetresonanzmessung Erzeugen mehrerer Pulszüge durch Aussenden mehrerer Anregungspulse zum Anregen von Kernspins in einem Patienten und Aussenden über die Körperspule (14);
(S40) Wiederholen des letzten Pulszuges der Magnetresonanzmessung, aufgrund dessen ersten Signalen und zweiten Signalen das Warnsignal erzeugt wurde, durch den Magnetresonanztomographen (1), wenn das Warnsignal von der Vorrichtung (60) eingeht,
wobei in Schritt (S20) Empfangen eines ersten Signals von der Lokalspule (50) über den ersten Signalanschluss (62) des ersten Empfängers (64) das erste Signal ein Magnetresonanzsignal aus dem Körper des Patienten ist.

12. Verfahren nach einem der Ansprüche 7 bis 10, wobei das Verfahren weiterhin die Schritte aufweist:
Während der Magnetresonanzmessung Erzeugen mehrerer Pulszüge durch Aussenden mehrerer Anregungspulse zum Anregen von Kernspins in einem Patienten und Aussenden über die Körperspule (14);
Entfernen des dem Spike entsprechenden Störsignals des letzten Pulszugs der Magnetresonanzmessung aufgrund dessen ersten Signalen und zweiten Signalen das Warnsignal erzeugt wurde, durch den Magnetresonanztomographen (1), wenn das Warnsignal von der Vorrichtung (60) eingeht,
wobei in Schritt (S20) Empfangen eines ersten Signals von der Lokalspule (50) über den ersten Signalanschluss (62) des ersten Empfängers (64) das erste Signal ein Magnetresonanzsignal aus dem Körper des Patienten ist.

13. Computerprogrammprodukt, welches direkt in einen Prozessor einer Steuerung (20) des Magnetresonanztomographen gemäß Anspruch 1 ladbar ist, mit Programmcode-Mitteln, um alle Schritte eines Verfahrens nach einem der Ansprüche 7 bis 12 auszuführen, wenn das Programmprodukt auf der Steuereinheit (20) ausgeführt wird.

14. Computerlesbares Speichermedium mit darauf gespeicherten elektronisch lesbaren Steuerinformationen, welche derart ausgestaltet sind, dass sie bei Verwendung des Speichermediums in der Steuereinheit (20) des Magnetresonanztomographen (1) gemäß Anspruch 1 das Verfahren nach einem der Ansprüche 7 bis 12 durchführen.

## Claims

1. Magnetic resonance tomograph having a device for detecting a spike during a magnetic resonance scan, wherein the device comprises:
a first receiver (64) with a first signal connection (62) for connecting a local coil (50) and a second receiver (65) with a second signal connection (63) for connecting a reference antenna,
wherein the device (60) is configured to compare a first signal received by the first receiver (64) via the first signal connection (62) with a second signal received by the second receiver (65) via the second signal connection (63), by means of a comparator unit (61) and to output a warning signal in the event of a predetermined deviation,
wherein the magnetic resonance tomograph comprises a body coil and a local coil, and the local coil is connected to the first signal connection,
**characterised in that**
the body coil is connected as a reference antenna to the second signal connection of the device.

2. Magnetic resonance tomograph according to claim **1,** wherein the device (60) is designed to carry out the comparison in real time.

3. Magnetic resonance tomograph according to claim 1 or 2, wherein the comparison comprises a pattern comparison between the first signal and the second signal.

4. Magnetic resonance tomograph according to one of the preceding claims, wherein the device (60) is configured only to carry out the comparison if the first and/or second signal fulfil a predetermined prior condition.

5. Magnetic resonance tomograph according to one of the preceding claims, wherein the predetermined deviation comprises an exceeding of a predetermined limit value for an amplitude ratio of the second signal to the first signal.

6. Magnetic resonance tomograph according to one of the preceding claims, having a control unit (20) in signal communication with the device, wherein the control unit (20) is designed, on arrival of the warning signal from the device (60), to repeat the sequence of an image acquisition after a last excitation pulse, on the basis of the first signals and second signals of which the warning signal was generated.

7. Method for recognizing a spike in a magnetic resonance scan with a magnetic resonance tomograph (1), wherein the magnetic resonance tomograph (1) comprises a local coil, a reference antenna and a device (60) for detecting a spike during a magnetic resonance scan with a first receiver (64) having a first signal connection (62) in signal communication with the local coil (50) and a second signal connection (63) in signal communication with the reference antenna,
wherein the magnetic resonance tomograph has a body coil, wherein the body coil is connected as a reference antenna to the second signal connection of the device,
wherein the method comprises the steps:
(S20) during a magnetic resonance scan, receiving a first signal from the local coil (50) via the first signal connection (62) of the first receiver (64);
(S21) receiving a second signal from the body coil via the second signal connection (63) of the second receiver (65);
(S30) comparing the first signal and the second signal by means of the device (60);
(S31) emission of a warning signal by the device (60) to the magnetic resonance tomograph (1) if during the comparison a predetermined deviation is detected.

8. Method according to claim 7, wherein the method further comprises the steps for calibration, wherein the steps for calibration are carried out before the steps in claim 7:
(S10) emission of a test pulse;
(S11) receiving a test signal via the local coil (50) and the first signal connection (62) of the device (60);
(S12) receiving the test signal via the reference antenna and the second signal connection (63) of the device (60);
(S13) determining a dependency of the test signal received via the first signal connection (62) on the test signal received via the second signal connection (63);
wherein the predetermined deviation in step (S31) is a substantial deviation of a dependency of the first signal on the second signal from the dependency determined in step (S13).

9. Method according to claim 8, wherein the dependency determined is a measure for the ratio of the amplitudes of the test signal received via the second signal connection (63) to the test signal received via the first signal connection (62) and a substantial deviation has taken place if the predetermined ratio is exceeded by more than 10%, 20%, 50% or 100%.

10. Method according to claim 8 or 9, wherein the step of receiving via the second signal connection (63) comprises the substep of detuning the body coil (14).

11. Method according to one of claims 7 to 10, wherein the method further comprises the steps:
during the magnetic resonance scan, generating multiple pulse trains by emitting multiple excitation pulses for exciting nuclear spins in a patient and emitting via the body coil (14); (S40) repeating the last pulse train of the magnetic resonance scan, on the basis of the first signals and second signals of which the warning signal was generated, by way of the magnetic resonance tomograph (1) if the warning signal arrives from the device (60),
wherein, in step (S20) receiving a first signal from the local coil (50) via the first signal connection (62) of the first receiver (64), the first signal is a magnetic resonance signal from the body of the patient.

12. Method according to one of claims 7 to 10, wherein the method further comprises the steps:
during the magnetic resonance scan, generating multiple pulse trains by emitting multiple excitation pulses for exciting nuclear spins in a patient and emitting via the body coil (14); removing the interference signal, which corresponds to the spike, of the last pulse train of the magnetic resonance scan, on the basis of the first signals and second signals of which the warning signal was generated, by way of the magnetic resonance tomograph (1) if the warning signal arrives from the device (60),
wherein, in step (S20) receiving a first signal from the local coil (50) via the first signal connection (62) of the first receiver (64), the first signal is a magnetic resonance signal from the body of the patient.

13. Computer program product which can be loaded directly into a processor of a control system (20) of the magnetic resonance tomograph according to claim 1, having program code means in order to carry out all the steps of a method according to one of claims 7 to 12 when the program product is executed on the control unit (20).

14. Computer readable storage medium with items of electronically readable control information stored thereon, which are configured in order, on use of the storage medium in the control unit (20) of the magnetic resonance tomograph (1) according to claim 1, to carry out the method according to one of claims 7 to 12.

## Revendications

1. Tomodensitomètre à résonance magnétique comprenant un dispositif de détection d'une spike pendant une mesure par résonance magnétique, dans lequel le dispositif comporte :
un premier récepteur (64) ayant une première borne (62) de signal pour la connexion d'une bobine (50) locale et un deuxième récepteur (65) ayant une deuxième borne (63) de signal pour la connexion d'une antenne de référence,
dans lequel le dispositif (60) est conçu pour comparer, au moyen d'une unité (61) de comparaison, un premier signal reçu par le premier récepteur (64) en passant par la première borne (62) de signal à un deuxième signal reçu par le deuxième récepteur (65) en passant par la deuxième borne (63) de signal et pour donner un signal d'alerte s'il y a un écart déterminé à l'avance,
dans lequel le tomodensitomètre à résonance magnétique a une bobine de corps et une bobine locale et la bobine locale est connectée à la première borne de signal,
**caractérisé en ce que** la bobine de corps est raccordée comme antenne de référence à la deuxième borne de signal du dispositif.

2. Tomodensitomètre à résonance magnétique suivant la revendication 1, dans lequel le dispositif (60) est conçu pour effectuer la comparaison en temps réel.

3. Tomodensitomètre à résonance magnétique suivant la revendication 1 ou 2, dans lequel la comparaison comporte une comparaison de configuration entre le premier signal et le deuxième signal.

4. Tomodensitomètre à résonance magnétique suivant l'une des revendications précédentes, dans lequel le dispositif (60) est conçu pour effectuer la comparaison, seulement si le premier et/ou le deuxième signal satisfont une condition préalable déterminée à l'avance.

5. Tomodensitomètre à résonance magnétique suivant l'une des revendications précédentes, dans lequel l'écart déterminé à l'avance comporte un dépassement, d'une valeur limite déterminée à l'avance, d'un rapport d'amplitude du deuxième signal au premier signal.

6. Tomodensitomètre à résonance magnétique suivant l'une des revendications précédentes, comprenant une unité (20) de commande en liaison de signal avec le dispositif, dans lequel l'unité (20) de commande est conçue pour, lors du lancement du signal d'alerte par le dispositif (60), répéter le déroulement d'une détection d'image après une dernière impulsion d'excitation, sur la base des premiers signaux et des deuxièmes signaux de laquelle le signal d'alerte a été produit.

7. Procédé de détection d'une spike dans une mesure par résonance magnétique par un tomodensitomètre (1) à résonance magnétique, dans lequel le tomodensitomètre (1) à résonance magnétique a une bobine locale, une antenne de référence et un dispositif (60) de détection d'une spike pendant une mesure par résonance magnétique, comprenant un premier récepteur (64) ayant une première borne (62) de signal en liaison de signal avec la bobine (50) locale et une deuxième borne (63) de signal en liaison de signal avec l'antenne de référence,
dans lequel le tomodensitomètre à résonance magnétique a une bobine de corps, dans lequel la bobine de corps est connectée comme antenne de référence à la deuxième borne de signal du dispositif,
dans lequel le procédé a les stades :
(S20) pendant une mesure par résonance magnétique, réception d'un premier signal par la bobine (50) locale en passant par la première borne (62) de signal du premier récepteur (64) ;
(S21) réception d'un deuxième signal par la bobine de corps en passant par la deuxième borne (63) de signal du deuxième récepteur (65) ;
(S30) comparaison du premier signal et du deuxième signal par le dispositif (60) ;
(S31) envoi d'un signal d'alerte par le dispositif (60) au tomodensitomètre (1) à résonance magnétique, si l'on détecte, à la comparaison, un écart déterminé à l'avance.

8. Procédé suivant la revendication 7, dans lequel le procédé a en outre les stades d'étalonnage, dans lequel on effectue les stades pour l'étalonnage avant les stades de la revendication 7 :
(S10) émission d'une impulsion de contrôle ;
(S11) réception d'un signal de contrôle par la bobine (50) locale et la première borne (62) de signal du dispositif (60) ;
(S12) réception du signal de contrôle par l'antenne de référence et par la deuxième borne (63) de signal du dispositif (60) ;
(S13) détermination d'une variation du signal de contrôle, reçu en passant par la première borne (62) de signal, par rapport au signal de contrôle, reçu en passant par la deuxième borne (63) de signal ;
dans lequel l'écart déterminé à l'avance dans le stade (S31) est un écart sensible d'une variation du premier signal par rapport au deuxième signal par rapport à la variation déterminée au stade (S13).

9. Procédé suivant la revendication 8, dans lequel la variation déterminée est une mesure du rapport des amplitudes du signal de contrôle reçu en passant par la deuxième borne (63) de signal au signal de contrôle reçu en passant par la première borne (62) de signal et il y a un écart sensible, si le rapport déterminé à l'avance est dépassé de plus de 10 %, 20 %, 50% ou 100 %.

10. Procédé suivant la revendication 8 ou 9, dans lequel le stade de la réception en passant par la deuxième borne (63) de signal comporte le stade partiel de désaccorder la bobine (14) de corps.

11. Procédé suivant l'une des revendications 7 à 10, dans lequel le procédé comporte en outre les stades :
pendant la mesure par résonance magnétique, production de plusieurs trains d'impulsions par envoi de plusieurs impulsions d'excitation pour l'excitation de spins de noyau dans un patient et envoi en passant par la bobine (14) de corps ;
(S40) répétition par le tomodensitomètre (1) à résonance magnétique du dernier train d'impulsions de la mesure par résonance magnétique,
sur la base des premiers signaux et des deuxièmes signaux duquel le signal d'alerte a été produit, si le signal d'alerte est lancé par le dispositif (60),
dans lequel, dans le stade (S20) réception d'un premier signal par la bobine (50) locale en passant par la première borne (62) de signal du premier récepteur (64), le premier signal est un signal de résonance magnétique provenant du corps du patient.

12. Procédé suivant l'une des revendications 7 à 10, dans lequel le procédé comporte en outre les stades :
pendant la mesure par résonance magnétique, production de plusieurs trains d'impulsions par envoi de plusieurs impulsions d'excitation pour l'excitation de spins de noyau dans un patient et envoi en passant par la bobine (14) de corps ;
élimination par le tomodensitomètre (1) à résonance magnétique du signal perturbateur, correspondant à la spike, du dernier train d'impulsions de la mesure par résonance magnétique sur la base des premiers et des deuxièmes signaux duquel le signal d'alerte a été produit, si le signal d'alerte est lancé par le dispositif (60),
dans lequel dans le stade (S20) de réception d'un premier signal par la bobine (50) locale en passant par la première borne (62) de signal du premier récepteur (64), le premier signal est un signal de résonance magnétique provenant du corps du patient.

13. Produit de programme d'ordinateur, qui peut être chargé directement dans un processeur d'une commande (20) du tomodensitomètre à résonance magnétique suivant la revendication 1, comprenant des moyens de code de programme pour exécuter tous les stades d'un procédé suivant l'une des revendications 7 à 12, lorsque le produit de programme est exécuté sur l'unité (20) de commande.

14. Support de mémoire déchiffrable par ordinateur, sur lequel sont mises en mémoire des informations de commande déchiffrables électroniquement, qui sont conformées de manière à ce qu'elles exécutent, lors de l'utilisation du support de mémoire dans l'unité (20) de commande du tomodensitomètre (1) à résonance magnétique suivant la revendication 1, le procédé suivant l'une des revendications 7 à 12.
